# EUROPEAN PATENT APPLICATION

(11) **EP 2 446 804 A1**
(43) Date of publication of application: **02.05.2012**
(21) Application number: 11767592.6
(22) Date of filing: 08.04.2011
(51) Int. Cl.: A61B 1/00

(54) **MEDICAL DEVICE**

(30) Priority: 13.07.2010 JP 2010158952
(71) Applicant: OLYMPUS MEDICAL SYSTEMS CORP., Tokyo 151-0072 (JP)
(72) Inventor: TAKAHASHI, Misa, Tokyo 151-0072 (JP); YAZAWA, Nobuyoshi, Tokyo 151-0072 (JP)
(74) Representative: Schicker, Silvia
(86) International application number: PCT/JP2011/058888
(87) International publication number: WO 2012/008188

(57) **Abstract**

A medical apparatus 1 to be introduced into a body 101, includes: image pickup means 50 for picking up an image in the body 101; a cable 43 extended from the image pickup apparatus 50, and configured to transmit and receive a signal or power to and from an external device 5, the cable being pulled out from inside to outside the body 101 through a body wall 102; an electric terminal portion 61 disposed at an end portion of the cable 43, which includes an electric contact 63 and is electrically connected to the external device 5; a cover body 60 which is configured to cover at least the electric contact 63 and to be adhered watertightly to the electric terminal portion 61 with a predetermined strength, the cover body being grasped when the cable 43 is pulled out from inside to outside the body 101; and an easy-peel portion 66 for detaching the cover body 60 from the electric terminal portion 61.

## Description

### Technical Field

The present invention relates to a medical apparatus provided with an image pickup apparatus which is used by being introduced in a body.

### Background Art

As is well known, endoscope apparatuses, which are medical apparatuses, include an image pickup apparatus in an endoscope and are configured to be introduced into a body cavity of a patient to carry out various kinds of inspection, treatment and the like of affected areas in the body based on observed images photographed by the image pickup apparatus.

As such endoscopes, in addition to a conventional type of endoscope which is introduced into digestive organs such as esophagus, stomach, large intestine, and duodenum, which are tube cavities in the body from the oral cavity or the anus, in recent years, attention has been drawn to an endoscope which is introduced into the abdominal cavity by being punctured from the vicinity of the umbilical region and penetrated through the body wall, for performing not only examination of digestive tracts but also a surgery for therapeutic treatment while observing organs in a body, what is called, a laparoscopic surgery. Such endoscope apparatuses include a cable for wired electrical connection between an endoscope to be introduced in a body and an external device such as a video processor.

Incidentally, some small medical cameras for observing inside a body cavity include a cable which is configured to transmit and receive video signals, control signals, signals for supplying power and the like, and the cable has an electric contact at the distal end portion thereof. In some cases, such a cable is once introduced in a body cavity together with a camera in order to fix the camera onto a body wall and then penetrated through the body wall to be extracted outside the body, and connected to a video processor as an external device. Therefore, it is necessary to provide a waterproof cap to the electric contact of the cable.

Such a cover body like a waterproof cap for waterproofing an electric contact portion of a cable when the cable is not connected to an external device has been conventionally proposed. For example, Japanese Patent Application Laid-Open Publication No. 2009-273792 discloses a waterproof cap which is attachable to and detachable from a connection port of a connector to which an electric contact member is disposed. The publication discloses a technology of an endoscope connector in which the conventional waterproof cap includes an O-ring which closely contacts the inner circumferential surface of the connection port to waterproof the connector and a sealing member which closely contacts the front face of a connector fixing/mounting rotation-ring arranged on the outer circumference of the connection port, for maintaining watertightness.

In addition, Japanese Patent Application Laid-Open Publication No. 10-199609, for example, discloses a technology of structure of a waterproof connector in which a slit is formed on a waterproof cap and the slit is engaged with a pin on a case in order to prevent a rotation torque from being applied to the internal connector when the outer circumference of a case placed for providing waterproof structure of the connector is grasped and rotated, thereby preventing the waterproof cap from rotating with respect to the case.

However, in the case of a medical apparatus whose cable is once introduced into a body cavity together with a camera, when the cable is extracted to the outside of the body by grasping the cover body such as a waterproof cap having a conventional structure, if the connection strength between the cover body and the connector is weak, there is a possibility that the cover body is detached from the connector in the body and dirt (mucosa, blood, etc.) adheres to an electric contact. On the other hand, if the cover body such as the waterproof cap put on the connector extracted to the outside of the body has a structure which is hard to be detached from the connector, it would take time to connect the connector to the video processor, and such a configuration is not suitable for a time-critical surgery.

Furthermore, in the case of the medical apparatus whose cable is once introduced into a body cavity together with the camera, since the cable passes through the body wall when the cable is extracted from the inside to the outside of the body, it is preferable to use the cover body covering the connector so as not to injure living body tissues.

Therefore, the present invention has been achieved in view of the above-described circumstances, and an object of the present invention is to provide a medical apparatus which is configured such that a cover body put on a connector disposed at an end portion of a cable has a structure hard to be detached from the connector for ensuring water-proofness and dirt-proofness and the like of an electric contact and the cover body can be easily detached from the connector when using the connector.

### Disclosure of Invention

### Means for Solving the Problem

A medical apparatus according to one aspect of the present invention is a medical apparatus configured to be introduced into a body, and includes: an image pickup apparatus for picking up an image in the body; a cable extended from the image pickup apparatus, and configured to transmit and receive a signal or power to and from an external device, the cable being pulled out from inside to outside the body through a body wall; an electric terminal portion disposed at an end portion of the cable, which includes an electric contact and is configured to be electrically connected to the external device; a cover body which is configured to cover at least the electric contact and to be adhered watertightly to the electric terminal portion with a predetermined strength, the cover body being grasped when the cable is pulled out from inside to outside the body; and an easy-peel portion for detaching the cover body from the electric terminal portion.

In addition, a medical apparatus according to another aspect of the present invention is a medical apparatus configured to be introduced into a body, and includes: image pickup means for picking up an image in the body; a cable extended from the image pickup means, and configured to transmit and receive a signal or power to and from an external device, the cable being pulled out from inside to outside the body through a body wall; an electric terminal portion disposed at an end portion of the cable, which includes an electric contact and is configured to be electrically connected to the external device; a cover body which is configured to cover at least the electric contact and to be adhered watertightly to the electric terminal portion with a predetermined strength, the cover body being grasped when the cable is pulled out from inside to outside the body; and easy-peel means for detaching the cover body from the electric terminal portion.

### Brief Description of the Drawings

FIG 1 relates to a first embodiment of the present invention and is a view showing a configuration of an endoscope system as a medical apparatus.
FIG 2 relates to the first embodiment of the present invention and is a cross-sectional view showing configurations of an extracorporeal apparatus and a puncture needle.
FIG 3 relates to the first embodiment of the present invention and is a cross-sectional view showing a configuration of an intra-abdominal cavity set camera.
FIG. 4 relates to the first embodiment of the present invention and is a perspective view showing a configuration of an end portion of an electric cable wire to which a drip-proof portion covering an electric terminal portion is mounted.
FIG 5 relates to the first embodiment of the present invention and is a perspective view showing a state where a ring-shaped latching portion of the drip-proof portion is hooked with a hook needle.
FIG 6 relates to the first embodiment of the present invention and is a perspective view showing a state at the time of detaching the drip-proof portion from the electric cable wire.
FIG. 7 relates to the first embodiment of the present invention and is a perspective view showing a state after the drip-proof portion was detached from the electric cable wire.
FIG. 8 relates to the first embodiment of the present invention and is a view showing a state where the hook needle is punctured in the abdomional wall and explaining a procedure for placing the intra-abdominal cavity set camera in the abdominal cavity.
FIG. 9 relates to the first embodiment of the present invention and is a view showing a state before a wire of the drip-proof portion is hooked with the hook needle and explaining a procedure for placing the intra-abdominal cavity set camera in the abdominal cavity.
FIG. 10 relates to the first embodiment of the present invention and is a cross-sectional view of the extracorporeal apparatus and the intra-abdominal cavity set camera in a state where the extracorporeal apparatus is arranged on the abdomen.
FIG. 11 relates to the first embodiment of the present invention and is a perspective view for illustrating a state where an electric terminal portion and the connector of the electric cable wire are to be connected.
FIG 12 relates to the first embodiment of the present invention and is an overall configurational view of the endoscope system which shows a state where the intra-abdominal cavity set camera is placed on the abdominal wall.
FIG. 13 relates to the first embodiment of the present invention and is a perspective view showing a configuration of a drip-proof portion according to a modified example.
FIG 14 relates to a second embodiment of the present invention and is a side view showing a configuration of a drip-proof portion.
FIG. 15 relates to the second embodiment of the present invention and is a cross-sectional view showing the configuration of the drip-proof portion in FIG 14.
FIG. 16 relates to the second embodiment of the present invention and is a side view showing a configuration of a drip-proof portion according to a modified example.
FIG. 17 relates to the second embodiment of the present invention and is a cross-sectional view showing the configuration of the drip-proof portion in FIG. 16.
FIG. 18 relates to the second embodiment of the present invention and is a side view showing a configuration in which an incision portion is provided on the drip-proof portion in FIG. 16.
FIG. 19 relates to the second embodiment of the present invention and is a view showing an example of a dedicated tool for detaching the drip-proof portion.
FIG. 20 relates to the second embodiment of the present invention and is a cross-sectional view showing the configuration of the dedicated tool for detaching the drip-proof portion in FIG. 19.
FIG. 21 relates to the second embodiment of the present invention and is a view showing an example of a dedicated tool for detaching the drip-proof portion, which is different from the one shown in FIG 19.
FIG 22 relates to the second embodiment of the present invention and is a plan view showing a configuration of a cutter unit in FIG. 21.
FIG 23 relates to a third embodiment of the present invention and is a side view showing a configuration of a drip-proof portion.
FIG. 24 relates to the third embodiment of the present invention and is a cross-sectional view showing the configuration of the drip-proof portion in FIG 23.
FIG. 25 relates to the third embodiment of the present invention and is a view showing a state where the drip-proof portion is dipped in medicinal solution.
FIG. 26 relates to the third embodiment of the present invention and is a view showing a state where the drip-proof portion is subjected to dipping processing in the medicinal solution.
FIG. 27 relates to the third embodiment of the present inventon and is a perspective view showing a configuration of an electric terminal portion according to a first modified example.
FIG. 28 relates to the third embodiment of the present invention and is a perspective view showing the configuration of the drip-proof portion covering the electric terminal portion in FIG. 27.
FIG. 29 relates to the third embodiment of the present invention and is a perspective view showing a configuration of an electric terminal portion according to a second modified example.
FIG. 30 relates to the third embodiment of the present invention and is an exploded perspective view showing the configuration of the drip-proof portion covering the electric terminal portion in FIG. 29.
FIG 31 relates to the third embodiment of the present invention and is a perspective view showing the configuration of the drip-proof portion covering the electric terminal portion in FIG 29.
FIG 32 relates to the third embodiment of the present invention and is a perspective view showing a modified example of an engaging portion.
FIG 33 relates to the third embodiment of the present invention and is a perspective view showing a configuration of a drip-proof portion covering an electric terminal portion according to a third modified example.

### Best Mode for Carrying Out the Invention

Hereinafter, a medical apparatus which is the present invention will be described. Note that, in the description hereinafter, each of the drawings according to each of the embodiments is a pattern diagram, and care should be taken to the fact that the relationship between the thicknesses and widths of the respective members, a ratio of the thickness of a certain component to others, and the like are different from the actual sizes. It is needless to say that the relationship and ratio among the dimensions of the components are sometimes different from one drawing to another. In addition, in the following description, a medical apparatus provided with a medical instrument for performing a laparoscopic surgery, for example.

### (First Embodiment)

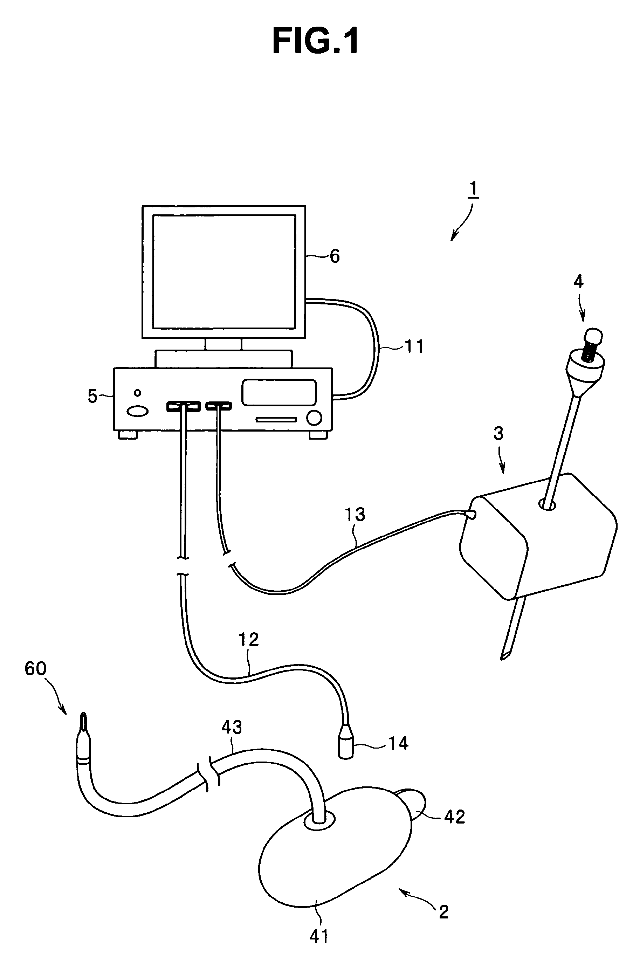

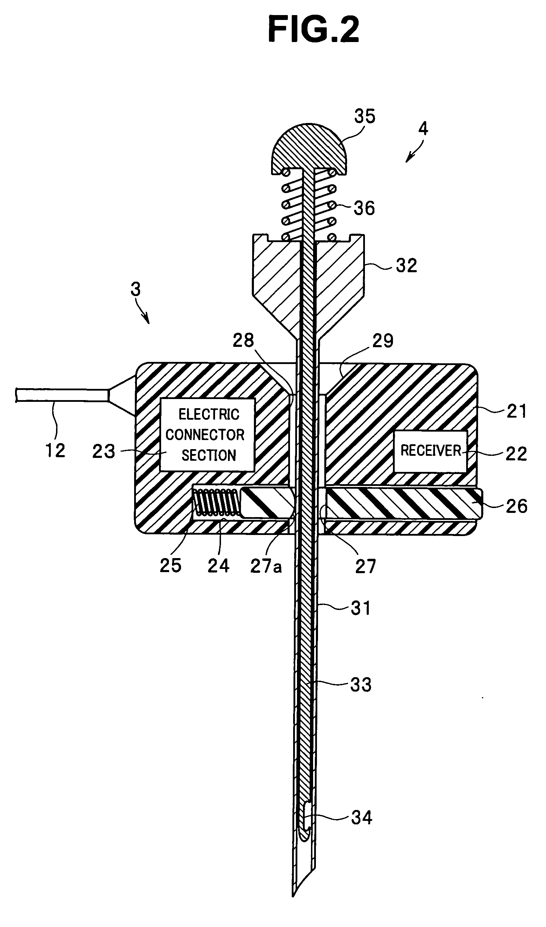

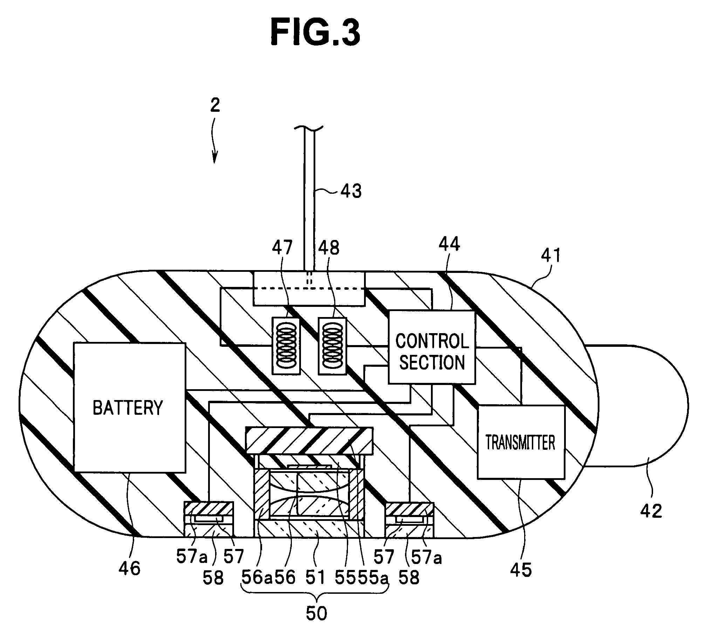

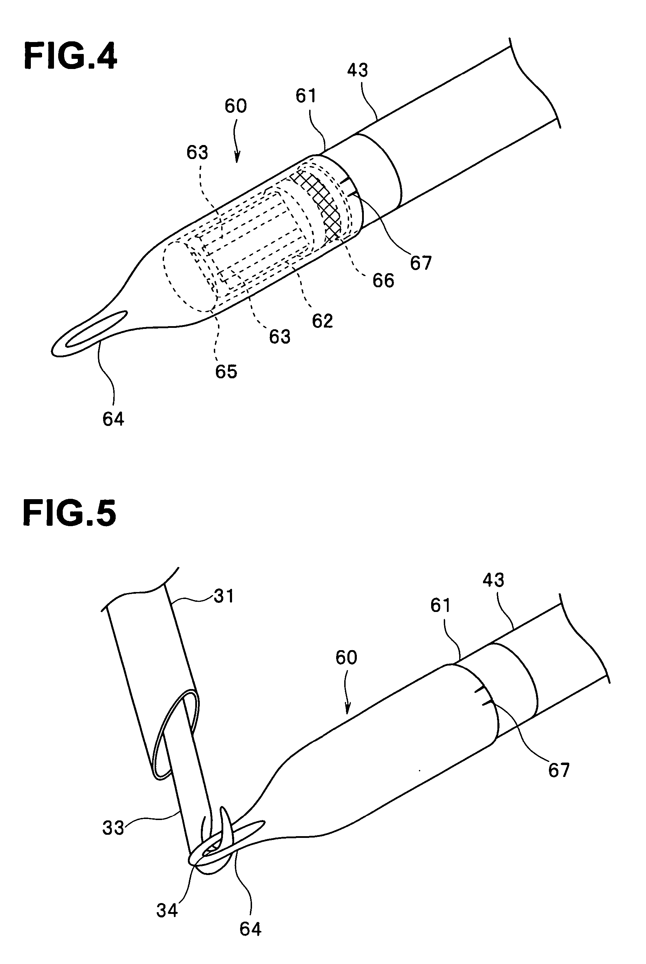

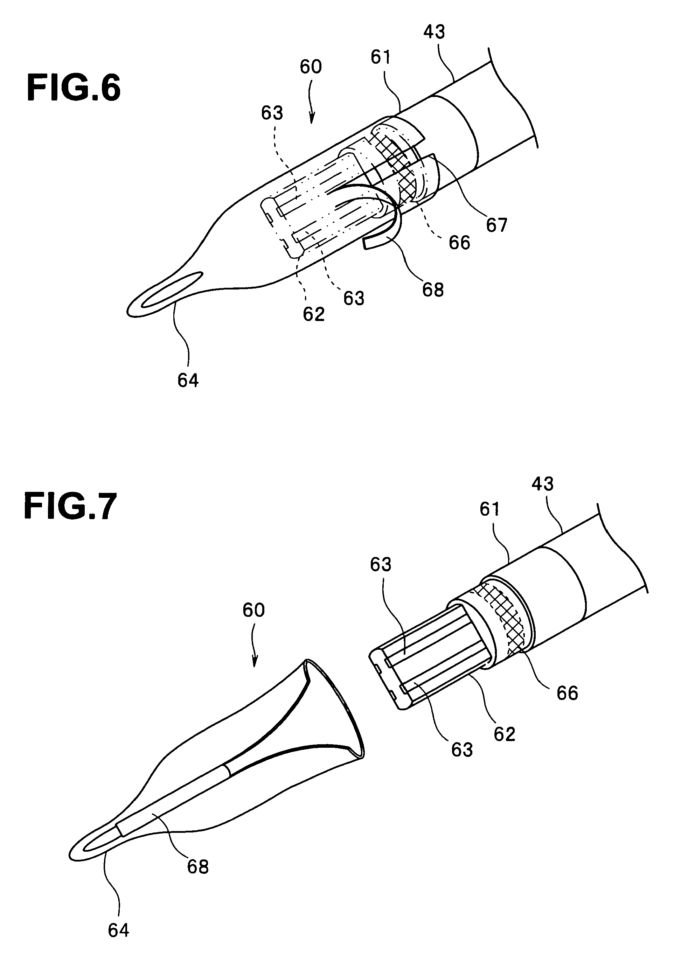

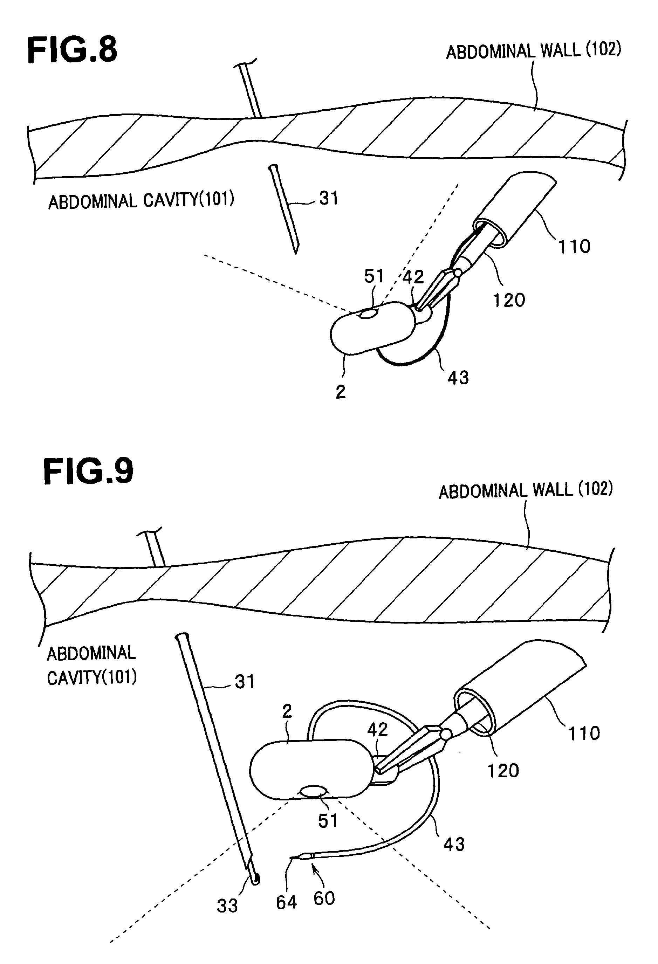

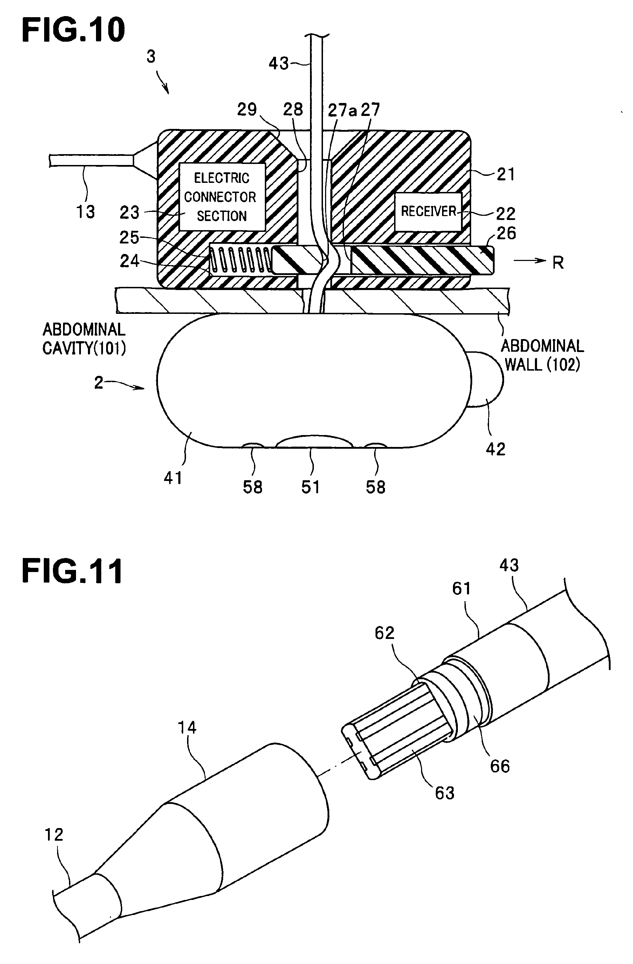

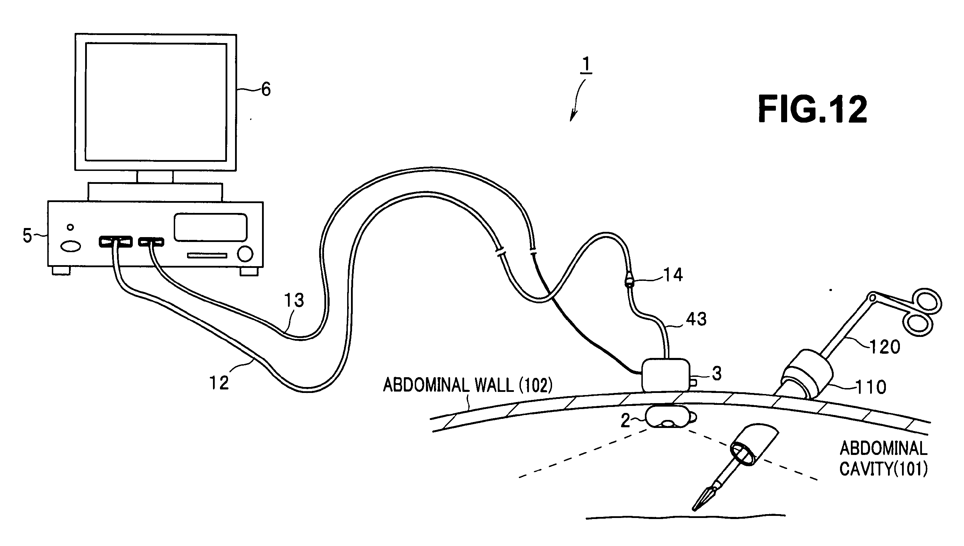

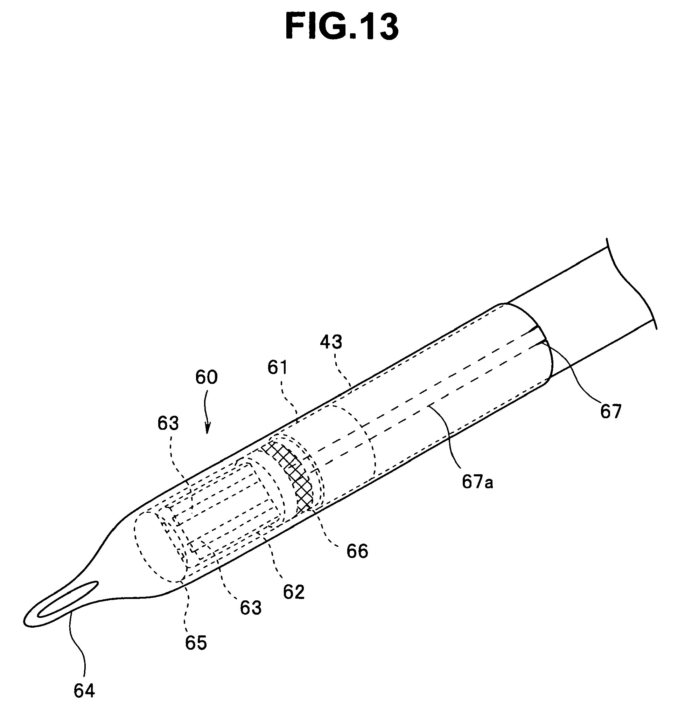

First, description will be made below on an endoscope system as a medical apparatus according to the present invention which is used for a laparoscopic surgery. Note that FIGS. 1 to 12 relate to the first embodiment of the present invention in which: FIG 1 is a view showing a configuration of an endoscope system as a medical apparatus; FIG. 2 is a cross-sectional view showing configurations of an extracorporeal apparatus and a puncture needle; FIG. 3 is a cross-sectional view showing a configuration of an intra-abdominal cavity set camera; FIG 4 is a perspective view showing a configuration of an end portion of an electric cable wire to which a drip-proof portion covering an electric terminal portion is mounted; FIG. 5 is a perspective view showing a state where a ring-shaped latching portion of the drip-proof portion is hooked with a hook needle; FIG 6 is a perspective view showing a state at the time of detaching the drip-proof portion from the electric cable wire; FIG 7 is a perspective view showing a state after the drip-proof portion was detached from the electric cable wire; FIG. 8 is a view showing a state where the hook needle is punctured in the abdominal wall and explaining a procedure for placing the intra-abdominal cavity set camera in the abdominal cavity; FIG 9 is a view showing a state before a wire of the drip-proof portion is hooked with the hook needle and explaining a procedure for placing the intra-abdominal cavity set camera in the abdominal cavity; FIG. 10 is a cross-sectional view of the extracorporeal apparatus and the intra-abdominal cavity set camera in a state where the extracorporeal apparatus is arranged on the abdomen; FIG 11 is a perspective view for illustrating a state where an electric terminal portion and the connector of the electric cable wire are to be connected; FIG 12 is an overall configurational view of the endoscope system which shows a state where the intra-abdominal cavity set camera is placed on the abdominal wall; and FIG. 13 is a perspective view showing a configuration of a drip-proof portion according to a modified example.

As shown in FIG. 1, an endoscope system 1 according to the present embodiment for performing a laparoscopic surgery is mainly configured by: an intra-abdominal cavity set camera (hereinafter, shortly referred to as camera) 2 which is a small capsule-type medical apparatus; an extracorporeal apparatus 3 as a camera fixing unit for fixing and holding the camera 2 introduced into a body from an extracorporeal side; a hook needle 4 as a puncture needle, which is insertable into and extractable from the extracorporeal apparatus 3, for hooking and pulling up the camera 2; a camera control unit (hereinafter abbreviated as CCU) as a signal processing apparatus in which an image processing circuit is incorporated; and a monitor 6 as a display apparatus which is electrically connected to the CCU 5 through a cable 11, for displaying observed images.

The CCU 5 is detachably connected with a communication cable 12 which is configured to be electrically connected to the camera 2, through an electric connector, and generates a video signal from an image signal transmitted from the camera 2 electrically connected to the communication cable 12, to output the generated video signal to the monitor 6. The monitor 6 is a liquid crystal display, for example, and receives the video signal outputted from the CCU 5 to display an observed image captured by the camera 2 on a screen. In addition, the CCU 5 is detachably connected to the extracorporeal apparatus 3 through an electric cable 13.

Next, the extracorporeal apparatus 3 and the hook needle 4 are detailed below with reference to FIG. 2.

As shown in FIG 2, the extracorporeal apparatus 3 is a camera fixing unit which pulls and fixes the camera 2 in a body cavity, and incorporates, in a housing 21, a receiver 22 and an electric connector section 23 which is electrically connected to the receiver 22.

The electric connector section 23 is connected to the electric cable 13 which is configured to be connected to the CCU 5. The extracorporeal apparatus 3 receives power supply from the CCU 5 and transmits signals from the receiver 22 to the CCU 5, through the electric cable 13.

The housing 21 has a slide hole portion 24 formed from a side surface in a lateral direction. In the slide hole portion 24, a fixing lever 26, which configures a fixing portion formed by a nonmagnetic material and having an end surface on which a biasing spring 25 is fixed, is arranged by insertion. The fixing lever 26 has substantially a rectangular parallelepiped shape, and is slidably disposed along the slide hole portion 24 toward the inner direction of the housing 21. In addition, the fixing lever 26 has a hole portion 27 formed at a midway portion thereof, and the hole portion 27 has a convex arc surface 27a on a side closer to the biasing spring 25.

The housing 21 has an insertion portion 28 penetrating vertically therethrough. The insertion portion 28 serves as an upper surface opening of the housing 21, and includes a conical tapered surface 29 formed so as to spread upward.

In the extracorporeal apparatus 3 configured as described above, the hook needle 4 configuring an end closing portion is insertably/extractably arranged in the hole which penetrates the extracorporeal apparatus 3 in the vertical direction when the fixing lever 26 is at a slide position where the fixing lever 26 is pushed into the housing 21 such that the hole portion 27 of the fixing lever 26 coincides with the insertion portion 28.

The hook needle 4 which is insertable into and extractable from the extracorporeal apparatus 3 includes: a cylindrical puncture needle tube 31; a needle head 32 provided in a linked manner on the upper portion of the puncture needle tube 31; a puncture rod 33 having at the distal end thereof a hook portion 34 and configured to be slidably inserted through the puncture needle tube 31; a hook head 35 provided in a linked manner on the upper portion of the puncture rod 33; and a spring 36 interposed between the hook head 35 and the needle head 32.

The puncture needle tube 31 is an elongated metal tube body, the diameter of which is approximately 3 millimeters, and has a distal end cut diagonally so as to form a sharp needle shape. The needle head 32 has an outer diameter larger than that of the puncture needle tube 31, and the distal end side of the needle head is formed in a conical shape integrally with the puncture needle tube 31. The needle head 32 abuts the tapered surface 29 formed at the upper portion of the housing 21, thereby preventing the hook needle 4 from falling off downward from the housing 21.

The puncture rod 33 is an elongated metal bar and the hook head 35 provided in a linked manner at the upper portion is biased in the direction apart from the needle head 32 by the spring 36. According to such a configuration, the puncture rod 33 is arranged such that the hook portion 34 formed at the distal end thereof is contained in the puncture needle tube 31.

In addition, the hook needle 4 is configured such that, when a user pushes the hook head 35 into the puncture needle tube 31 against the biasing force of the spring 36, the hook portion 34 formed at the distal end of the hook needle protrudes from the distal end of the puncture needle tube 31.

The hook needle 4 thus configured is inserted and fixed in the housing 21 by a pressing force in the outward direction of the housing 21 generated by the fixing lever 26 being biased by the biasing force of the biasing spring 25, in the state inserted and arranged in the insertion portion 28 of the housing 21 and the hole portion 27 of the fixing lever 26. That is, the hook needle 4 is fixed in the state inserted in the housing 21 by the outer circumferential surface of the puncture needle tube 31 being pressed by the arc surface 27a formed on one side surface of the hole portion 27 of the fixing lever 26 and abutting the inner surface of the insertion portion 28.

Next, the camera 2 will be detailed below with reference to FIG. 3.

The camera 2 includes, as shown in FIG 3, an exterior part 41 configuring a capsule-type housing part, a plate-like grasping portion 42 protruded from one side portion of the exterior part 41, and a communication cable 43 provided so as to be extended from a center of the upper part of the exterior part 41.

The exterior part 41 includes inside thereof a control section 44, a transmitter 45, a battery 46, and two electromagnetic coils 47, 48, an image pickup unit 50 which configures a camera unit as an image pickup section and includes inside thereof image pickup means, and a plurality of illumination sections 57 which configure a compact and low-power-consumption illumination unit configured by LEDs, organic ELs, or the like, as a light source of illumination light.

The control section 44 is electrically connected to the transmitter 45, the image pickup unit 50, and the illumination sections 57. Note that the control section 44 receives driving power supply through the battery 46 or the communication cable 43, for controlling and driving each of the components. In addition, the control section 44 is electrically connected also to the electromagnetic coil 48 which is one of the electromagnetic coils. The other electromagnetic coil 47 is connected with one of the wirings inserted through the communication cable 43.

The image pickup unit 50 is configured by mainly including a solid-state image pickup device 55 such as a CCD, a CMOS, or the like, an image pickup device driving circuit section 55a for driving and controlling the solid-state image pickup device 55 and photoelectrically converting the photographing light incident on the solid-state image pickup device 55, an objective lens group 56 for condensing the photographing light onto the solid-state image pickup device 55, and a lens holding frame 56a for holding the objective lens group 56. Note that, in the image pickup unit 50 according to the present embodiment, an image pickup optical system which allows an image pickup over a wide-angle field of view is set such that the photographable angle of view is equal to or larger than 120 degrees, for example. In addition, the image pickup unit 50 may include a zooming function and the like.

Each of the illumination sections 57 is arranged on an illumination driving circuit section 57a with which the illumination section is driven. Note that the exterior part 41 includes covering members 51, 58 made of a sapphire glass and the like and configuring a transparent observation window and illumination windows, for airtightly holding and covering the image pickup unit 50 and the respective illumination sections 57. The covering members 51, 58 are arranged at the lower portion of the exterior part 41.

In addition, in the camera 2, when power is supplied to the communication cable 43, current is supplied to the electromagnetic coil 47, and the magnetic force generated in the electromagnetic coil 47 causes inductive current to be generated in the other electromagnetic coil 48, and the generated inductive current is inputted to the control section 44. The control section 44 includes inside thereof a changeover switch, not shown, and is configured to operate the internal switch such that the power supply from the battery 46 is switched to the power supply through the communication cable 43, upon receiving the inductive current from the electromagnetic coil 48.

Note that the communication cable 43 is a communication cable for transmitting and receiving various instruction signals between the CCU 5 and the control section 44 in the camera 2, and for transmitting and receiving image pickup signals between the image pickup unit 50 and the CCU 5.

Next, detailed description will be made on an electric terminal portion provided at an extension end of the communication cable 43 extended from the exterior part 41 of the camera 2, and a cover body configured to cover the electric terminal portion, with reference to FIGS. 4 to 7.

As shown in FIGS. 4, 6 and 7, the communication cable 43 includes at an extension end thereof an electric terminal portion 61. The electric terminal portion 61 is a male connector including a male terminal 62 having a substantially rectangular block shape and formed so as to be extended forward. The male terminal 62 is configured to be electrically connectable to a female connector 14 (see FIG. 1) provided at the end portion of the communication cable 12 connected to the CCU 5.

In addition, the male terminal 62 of the electric terminal portion 61 includes on both side surfaces thereof a plurality of electric contacts 63. A drip-proof portion (also referred to as waterproof cap) 60 as a cover body for waterproof and dirt prevention is mounted on the electric terminal portion 61 so as to cover the male terminal 62. The male terminal 62 is thus covered with the drip-proof portion 60 mounted thereon, to be drip-proofed (prevented from adherence of dirt such as body fluid and blood, and prevented from dust).

As shown in FIG. 5, the drip-proof portion 60 integrally includes, on a forward side thereof, a latching portion 64 formed in a loop shape so as to be able to be grasped by hooking the hook portion 34 of the hook needle 4, and is thinly formed as a cylindrical film whose front end is closed, by using an easy-peel (easy-to-unseal) resin (also referred to as easy-sealant member, and materials such as polypropylene and polyethylene). Furthermore, the drip-proof portion 60 includes a space portion 65 for housing the male terminal 62 of the electric terminal portion 61, so as to cover the plurality of electric contacts 63.

Therefore, the drip-proof portion 60 is disposed so as to cover the entire of the male terminal 62 and the distal end outer circumferential part of the electric terminal portion 61, and configures a waterproof and dirt proof cover body for watertightly holding the male terminal 62. Note that the drip-proof portion 60 has a shape smoothly sloping toward rearward, with the front side latching portion 64 formed in a tapered shape. It is preferable that the above-described easy-peel resin has a biocompatibility and high heat resistance and slidability.

In the drip-proof portion 60 as described above, a part of the inner circumference thereof is formed by a seal 66, as an easy-peel adhering portion which serves as easy-peel means (easy-peel portion), subjected to adhesive processing such as thermal bonding and pressure bonding so as to be bonded with the rear outer circumferential portion of the male terminal 62, thereby enabling cohesion peeling, interlaminar peeling, and interfacial peeling of the bonded part to be easily performed from a predetermined direction by taking advantage of the characteristic of the easy-peel resin.

The bonded part between the part of the inner circumference of the drip-proof portion 60 and the rear outer circumferential portion of the male terminal 62 has a sufficient strength not to be easily come off, even if the drip-proof portion is pulled with a certain amount of force by hooking the hook portion 34 of the hook needle 4 in the latching portion 64. In addition, the drip-proof portion 60 has, at a part of the proximal end peripheral portion thereof, two incisions 67 formed in the longitudinal direction, which configure an easy-to-unseal processed part, in the present embodiment, and the drip-proof portion 60 can be easily detached from the electric terminal portion 61 by tearing a peelable strip 68 forward in a strip shape along the incisions 67 to peel off a part of the seal 66, and thereafter, by peeling the drip-proof portion 60 off along the circumferential direction of the seal 66. The incisions 67 also configure one of easy-to-peel means.

Note that separation means provided at the proximal end part of the drip-proof portion 60 is not limited to the incisions 67, and the easy-to-unseal technology Magic Cut (Registered Trademark) processing may be adopted as easy-to-peel means for enabling the drip-proof portion to be torn off from any position of the proximal end part. In addition, the drip-proof portion 60 may be provided with easy-to-unseal processed perforations, as easy-to-peel means, formed finely along the incisions 67 so as not to allow liquids to enter inside the drip-proof portion, in order to enable the peelable strip 68 to be easily torn off in a strip shape.

The above-configured endoscope system 1 according to the present embodiment is used for a laparoscopic surgery for treatment inside the abdominal cavity which is one of the body cavities of a patient.

Here, detailed description will be made below regarding a procedure and working for setting the camera 2 of the endoscope system 1 according to the present embodiment in the abdominal cavity as a body cavity of the patient for a laparoscopic surgery, with reference to drawings, mainly, FIGS. 8 to 12.

First, as shown in FIG. 2, an operator inserts the puncture needle tube 31 of the hook needle 4 into the insertion portion 28 provided to the extracorporeal apparatus 3. At this time, the operator pushes the fixing lever 26 into the housing 21 to allow the puncture needle tube 31 to penetrate through the extracorporeal apparatus 3, and inserts the puncture needle tube 31 so as to penetrate the hole portion 27 of the fixing lever 26. In addition, the extracorporeal apparatus 3 is in a state connected to the CCU 5 through the electric connector of the electric cable 13.

Then, the operator allows the puncture needle tube 31 to protrude sufficiently from the under surface of the extracorporeal apparatus 3 in a state where the extracorporeal apparatus 3 is located sufficiently close to the needle head 32 side which is a hand side of the puncture needle tube 31 (See FIGS. 2 and 3). In this state, the extracorporeal apparatus 3 is prevented from being fallen off from the puncture needle tube 31, by the arc surface 27a, which is one wall surface of the hole portion 27 of the fixing lever 26, abutting and pressing the puncture needle tube 31 by the biasing force of the biasing spring 25 acting on the fixing lever 26.

Next, the operator forms a small incision site on the abdominal wall 102 of the patient with a surgical knife and the like, and punctures a trocar 110 into the incision site. Then, the operator grasps a grasping portion 42 of the camera 2 with a treatment instrument 120 such as a grasping forceps, to introduce the camera 2 into the abdominal cavity 101 through the trocar 110 punctured in the abdominal wall 102. Note that the operator mounts the drip-proof portion 60, in advance, onto the electric terminal portion 61 of the communication cable 43 of the camera 2.

After introducing the camera 2 into the abdominal cavity 101, as shown in FIGS. 8 and 9, the operator directs the observation direction of the camera 2 in a direction for enabling the wall surface direction of the abdominal wall 102 in the abdominal cavity 101 to be photographed. Then, the operator punctures the puncture needle tube 31 of the hook needle 4 which is inserted into and held by the extracorporeal apparatus 3 so as to penetrate through the abdominal wall 102, while checking an image photographed by the camera 2 on the monitor 6. At this time, the camera 2 is driven by the power supply from the battery 46, and wirelessly transmits image signals picked up by the image pickup unit 50 from the transmitter 45 to the receiver 22 of the extracorporeal apparatus 3.

After that, the operator pushes the hook head 35 for allowing the puncture rod 33 to be pushed out from the puncture needle tube 31 (see FIG. 2). At this time, the operator changes the observation direction of the camera 2, as shown in FIG. 9, to check the position of the latching portion 64 of the drip-proof portion 60 mounted onto the end portion of the communication cable 43. Note that FIG 9 shows the state where the camera 2 photographing the upper side, i.e., the direction of the abdominal wall 102 was turned around and the lower side is being photographed.

The operator hooks the hook portion 34 formed at the puncture rod 33 in the latching portion 64 of the drip-proof portion 60 while checking the observed image by the camera 2 displayed on the monitor 6 (see FIG 5). When the latching portion 64 of the drip-proof portion 60 is hooked on the hook portion 34, the operator releases the pushing of the hook head 35 of the puncture rod 33. Then, the puncture rod 33 is introduced into the puncture needle tube 31 with the latching portion 64 hooked on the hook portion 34.

Next, the operator extracts the puncture needle tube 31 of the hook needle 4 from the abdominal cavity 101 to outside the body with the latching portion 64 of the drip-proof portion 60 hooked on the hook portion 34 of the puncture rod 33. Furthermore, the operator further pulls the hook needle 4 to the outside the body such that the communication cable 43 on which the drip-proof portion 60 is mounted penetrates through the abdominal wall 102 and extends to the outside the body.

Note that the drip-proof portion 60 has a shape smoothly sloping toward rearward, with the front side latching portion 64 formed in a tapered shape. Therefore, when passing through the abdominal wall 102, the drip-proof portion 60 can easily pass through the abdominal wall 102 without damaging the living tissue of the abdominal wall 102. In addition, the drip-proof portion 60 has a bond strength not to be easily detached from the electric terminal portion 61 even the amount of force to the extent that the communication cable 43 is penetrated through the abdominal wall 102 and extracted to the outside the body is applied. Therefore, it is not necessary for the user to adjust or give attention to the amount of pulling force.

Then, the operator pulls the communication cable 43 to move the extracorporeal apparatus 3 relatively with respect to the puncture needle tube 31 in the abdominal direction of the patient, and pulls the puncture needle tube 31 until the communication cable 43 on which the drip-proof portion 60 is mounted penetrates through the insertion portion 28 of the extracorporeal apparatus 3.

At this time, the operator pushes the fixing lever 26 of the extracorporeal apparatus 3 to the inside of the housing 21, thereby capable of easily sliding the extracorporeal apparatus 3 relatively with respect to the puncture needle tube 31 of the hook needle 4. When the communication cable 43 penetrates through the insertion portion 28 of the extracorporeal apparatus 3, the operator moves the extracorporeal apparatus 3 in the abdominal direction of the patient along the communication cable 43 this time, while pulling the communication cable 43.

That is, the operator can easily slide the extracorporeal apparatus 3 relatively with respect to the communication cable 43 by maintaining the state in which the fixing lever 26 of the extracorporeal apparatus 3 is pushed to the inside of the housing 21.

Then, as shown in FIG 10, the operator pulls the communication cable 43 until the abdominal wall 102 is brought into a state sandwiched between the extracorporeal apparatus 3 and the camera 2, with the extracorporeal apparatus 3 placed on the abdomen of the patient. At this time, the operator brings the upper portion of the camera 2 into sufficiently close contact with the inner surface of the abdominal wall 102 and releases the pushing of the fixing lever 26 of the extracorporeal apparatus 3.

The fixing lever 26 of the extracorporeal apparatus 3 receives the biasing force of the biasing spring 25 to move in the arrow R direction, which brings the hole portion 27 into a state displaced from the insertion portion 28 of the housing 21. The communication cable 43 inserted through the hole portion 27 and the insertion portion 28 is brought into a sandwiched state, to be engaged by the housing 21. At this time, the communication cable 43 is constantly subjected to a tensional force of a certain level or higher. Accordingly, the state where the extracorporeal apparatus 3 and the camera 2 sandwich the abdominal wall 102 therebetween is maintained by the tensional force of a certain level or higher constantly applied to the communication cable 43 being maintained, which allows the camera 2 to be fixed onto the inner surface of the abdominal wall 102 in the abdominal cavity 101.

Then, as shown in FIG 6 and FIG. 7, the operator detaches the drip-proof portion 60 mounted at the end of the communication cable 43 pulled out to the outside the body. At this time, the operator peels off the seal 66 of the drip-proof portion 60 which is adhered to the male terminal 62 by taking advantage of the characteristic of the easy-peel resin, thereby capable of easily detaching the drip-proof portion 60 from the electric terminal portion 61. Finally, the operator connects the electric terminal portion 61 of the communication cable 43 to the connector 14 of the communication cable 12 connected to the CCU 5 (see FIG. 11).

Then, in the camera 2, current is supplied from the communication cable 43 to the electromagnetic coil 47 shown in FIG. 3. A magnetic force is generated in the electromagnetic coil 47 by the current flowing therethrough, and the inductive current generated in the other electromagnetic coil 48 is inputted to the control section 44.

The control section 44 receives the inductive current generated in the electromagnetic coil 48 to switch the internal changeover switch, thereby switching the power supply from the battery 46 to the power supply from the communication cable 43.
As shown in FIG 12, the camera 2 is firmly placed in the abdominal cavity 101 of the patient in a stable state, and a laparoscopic surgery is performed by the endoscope system 1 according to the present embodiment. Note that one end portion of a pneumoperitoneum tube, not shown, is mounted to the trocar 110, for example, and carbon dioxide gas, for example, is injected in the abdominal cavity 101, as pneumoperitoneum gas for the purpose of ensuring a field of view of the camera 2 and ensuring a region for operating a surgical instrument and the like which is to be introduced into the abdominal cavity 101. The operator performs a laparoscopic surgery by inserting the treatment instrument 120 into the trocar 110 in the state where the camera 2 is pulled up to the abdominal wall 102 and placed in the abdominal cavity 101.
Note that when finishing the laparoscopic surgery, the operator pulls out the extracorporeal apparatus 3 from the latching portion 64 of the drip-proof portion 60 while pushing the fixing lever 26 of the extracorporeal apparatus 3 to the inside of the housing 21. Then, the operator grasps the camera 2 in the abdominal cavity 101 with the treatment instrument 120 such as a grasping forceps, to take out the camera 2 to the outside the body through the trocar 110.
According to the endoscope system 1 of the above-described present embodiment, when the camera 2 is introduced into the abdominal cavity 101, the electric terminal portion 61 of the communication cable 43 which transmits various signals from the camera 2 by wire is covered with the drip-proof portion 60, so that there is no need for exposing the electric contacts 63 of the electric terminal portion 61 in the abdominal cavity 101. Therefore, the electric contacts 63 are not exposed in the humid abdominal cavity 101, which prevents adherence of dirt such as blood and body fluid and suppresses occurrence of degradation, corrosion and the like.
In addition, in order to improve the operability in the abdominal cavity 101 as a limited space when the camera 2 is introduced into the abdominal cavity 101 and the communication cable 43 is taken out from inside of the abdominal cavity 101 to the outside the body, the drip-proof portion 60 is provided with the loop-shaped latching portion 64 so that the hook portion 34 formed at the distal end of the puncture rod 33 of the hook needle 4 can be easily hooked. The user as an operator can easily pull out the communication cable 43 of the camera 2 from the abdominal cavity 101 to the outside the body, by mounting the drip-proof portion 60 onto the electric terminal portion 61 of the communication cable 43, which improves workability of the medical procedure.
Furthermore, the user as the operator peels off the seal 66 adhering the drip-proof portion 60 and the electric terminal portion 61, by tearing along the two incisions 67, thereby capable of easily detaching the drip-proof portion 60 from the electric terminal portion 61. The user as the operator can immediately connect the electric terminal portion 61 to the connector 14 of the communication cable 12.
As described above, the endoscope system 1 according to the present embodiment is capable of surely waterproofing the electric contacts 63 and preventing adhesion of dirt, by providing the seal 66 having an adhesion structure with which the drip-proof portion 60 is hardly detached from the electric terminal portion 61 even if the drip-proof portion 60, as a cover body, fitted on the electric terminal portion 61 disposed at the end portion of the communication cable 43 is pulled with a predetermined amount of force, and also capable of improving the workability of the medical procedure by the user due to the configuration enabling the drip-proof portion 60 to be immediately detached from the electric terminal portion 61 when using the system.

### (First Modified Example)

As shown in FIG. 13, the drip-proof portion 60 may be configured to cover up to the midway portion of the communication cable 43. Furthermore, the drip-proof portion 60 may be configured to cover the entire of the communication cable 43.

According to such a configuration, dirt is prevented from adhering on the part of the communication cable 43 covered with the drip-proof portion 60, which enables the outer surface part of the communication cable 43 covered with the drip-proof portion 60 to be used in a clean state by detaching the drip-proof portion 60.

In addition, the drip-proof portion 60 is provided with perforations 67a, as an easy-to-unseal processed part, formed along the incisions 67 such that the peelable strip 68 can be easily torn off, which enables the drip-proof portion 60 to be easily detached from the communication cable 43.

### (Second Embodiment)

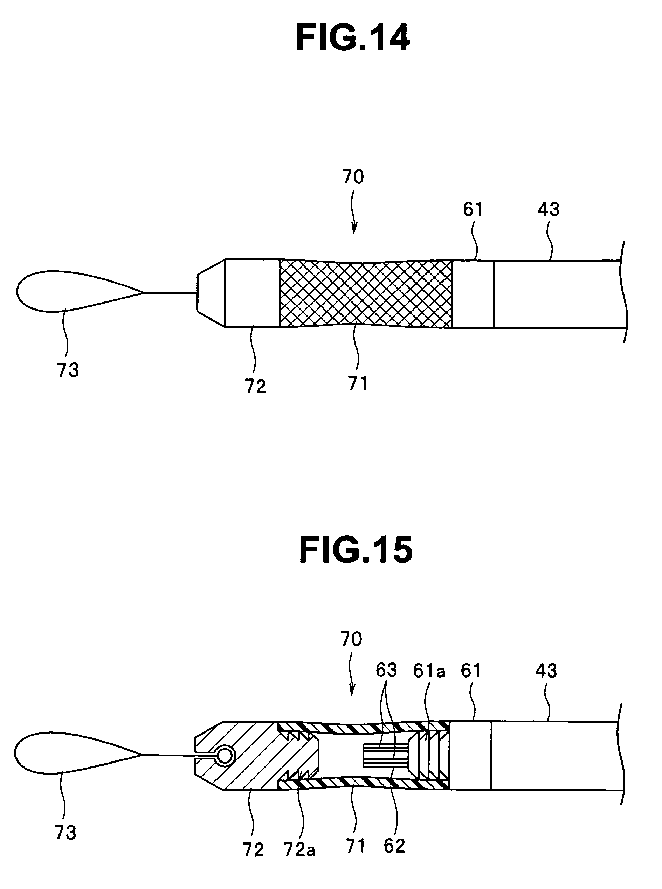

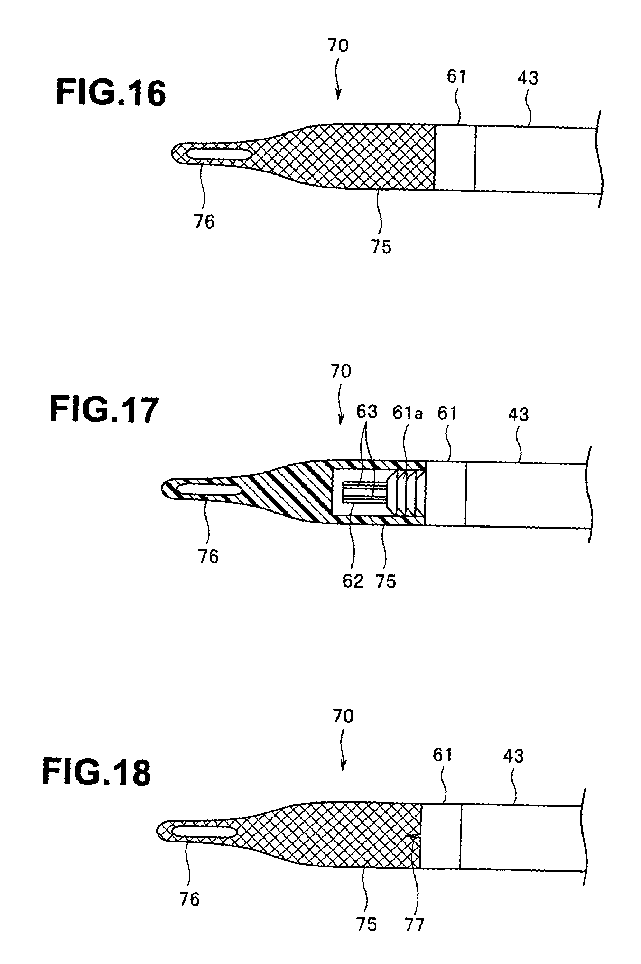

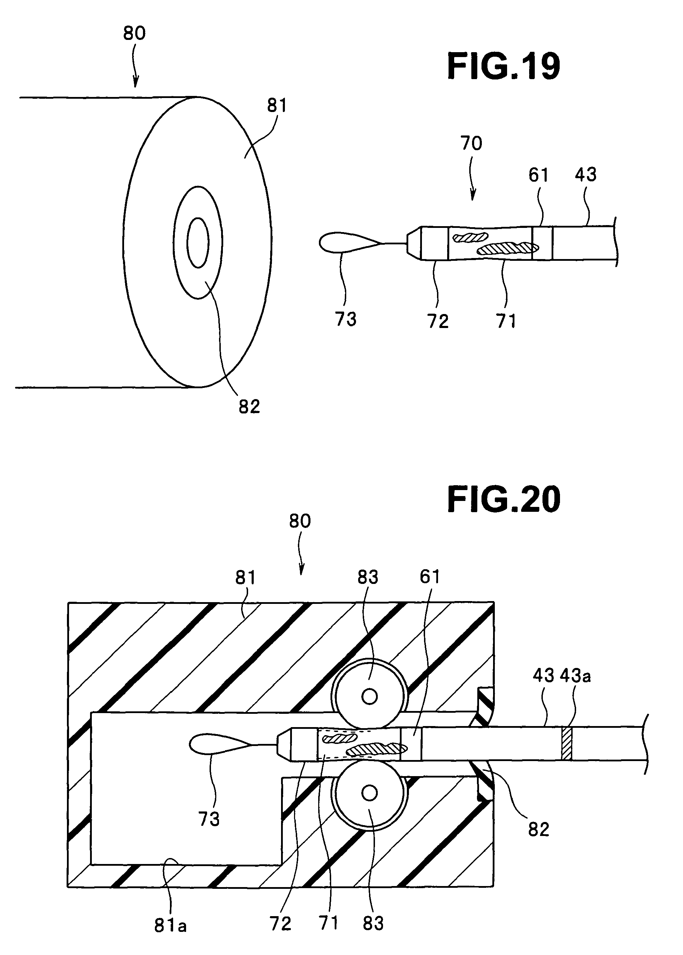

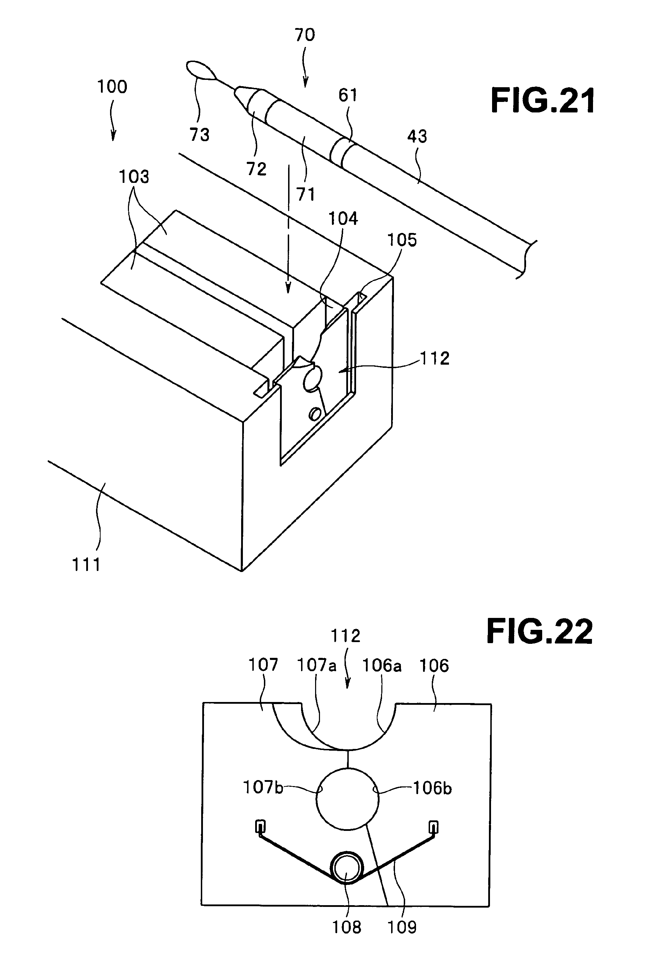

Next, with reference to FIGS. 14 to 20, the second embodiment of the endoscope system of the present invention will be described below. Note that, FIGS. 14 to 20 relate to the second embodiment of the present invention, in which: FIG. 14 is a side view showing a configuration of a drip-proof portion; FIG 15 is a cross-sectional view showing the configuration of the drip-proof portion in FIG. 14; FIG. 16 is a side view showing a configuration of a drip-proof portion according to a modified example; FIG. 17 is a cross-sectional view showing the configuration of the drip-proof portion in FIG. 16; FIG. 18 is a side view showing a configuration in which an incision portion is provided on the drip-proof portion in FIG. 16; FIG. 19 is a view showing an example of a dedicated tool for detaching the drip-proof portion; FIG. 20 is a cross-sectional view showing the configuration of the dedicated tool for detaching the drip-proof portion shown in FIG. 19; FIG. 21 is a view showing an example of a dedicated tool for detaching the drip-proof portion, which is different from the one shown in FIG. 19; and FIG. 22 is a plan view showing a configuration of a cutter unit in FIG. 21. In addition, in the description below, the same reference numerals are attached to the same components as those in the endoscope system 1 according to the first embodiment, detailed descriptions thereof will be omitted, and the features thereof can be applied to the first embodiment.

As shown in FIGS. 14 and 15, a drip-proof portion as a cover body (drip-proof portion) 70 according to the present embodiment includes a cylindrical heat-shrinkable tube 71, and a head portion 72 including on the front side thereof a latching portion 73 formed by a loop-shaped wire. Note that the latching portion 73 is configured to hold the hook portion 34 of the hook needle 4 by hooking, similarly as in the first embodiment.

The heat-shrinkable tube 71 of the drip-proof portion 70 has a rearward opening portion in which the distal end part of the electric terminal portion 61 including the male terminal 62 is inserted, and has a forward opening portion into which the rear part of the head portion 72 is inserted, and the heat-shrinkable tube 71 is shrunk by heat processing. The heat-shrinkable tube 71 watertightly contains the male terminal 62 of the electric terminal portion 61 so as to cover a plurality of electric contacts 63.

Note that the electric terminal portion 61 and the head portion 72 covered with the heat-shrinkable tube 71 have so-called tube fitting structures 61a, 72a which are formed so as to be projected and recessed in a circumferential direction. The tube fitting structures 61 a, 72a are thus provided, the electric terminal portion 61 and the head portion 72 connected through the heat-shrinkable tube 71 have strength not to be easily detached from each other even when pulled in the longitudinal direction with a certain amount of force.

In addition, the head portion 72 has a taper in which the front part is tapered and smoothly sloping toward rearward, which allows the drip-proof portion 70 to easily pass through the abdominal wall 102 without damaging the living tissue of the abdominal wall 102 when passing through the abdominal wall 102.

The above-configured drip-proof portion 70 according to the present embodiment can be reduced in diameter by use of the heat-shrinkable tube 71 without increasing the size of the outer diameter, in addition to the effects described in the first embodiment.

Note that, as shown in FIGS. 16 and 17, the drip-proof portion 70 may be a heat-shrinkable body (tube) 75 having a loop-shaped latching portion 76 formed integrally at the front part. Furthermore, as shown in FIG. 18, an incision 77, as an easy-to-unseal processed part, for facilitating detachment of the drip-proof portion 70 from the electric terminal portion 61, may be formed in the longitudinal direction at a part of the proximal end of the drip-proof portion 70.

Incidentally, a dedicated detachment tool 80 configuring easy-peel means (easy-peel portion) as shown in FIGS. 19 and 20 is used for enabling the drip-proof portion 70 to be easily detached from the electric terminal portion 61.

The dedicated detachment tool 80 includes a valve 82 for dirt removal formed on one surface of a housing 81, and the communication cable 43 on which the drip-proof portion 70 is mounted is inserted from an opening portion of the valve 82. Note that the valve 82 is formed by rubber, sponge and the like, for removing dirt adhered on the drip-proof portion 70 and the distal end part of the communication cable 43.

When the drip-proof portion 70 mounted on the communication cable 43 is inserted in the dedicated detachment tool 80, two disk-shaped cutters 83 provided inside the tool rotate, and incisions (shown by dashed lines in FIG. 20) are formed on the heat-shrinkable tube 71. When the incisions are formed on the entire length of the heat-shrinkable tube 71, the heat-shrinkable tube 71 is separated into two parts to be detached from the electric terminal portion 61 and fallen off, together with the head portion 72 having the latching portion 73, in a space 81 a in the housing 81. Then, if the communication cable 43 is pulled out from the dedicated detachment tool 80, the drip-proof portion 70 can be easily detached and the electric terminal portion 61 can be exposed.

In addition, the communication cable 43 is provided with a marking 43a formed at a position a predetermined length away from the electric terminal portion 61 so that the incisions can be surely formed on the entire length of the heat-shrinkable tube 71 by the two disk-shaped cutters 83, which provides an indication of insertion length of the communication cable 43 into the dedicated detachment tool 80.

In addition, as shown in FIGS. 21 and 22, the dedicated detachment tool may be configured as a dedicated detachment tool 100 for detaching the easy-peel means, which is provided with a cutter unit 112 having two blades 106 and 107 which are configured to rotate relatively with respect to each other.

For details, the dedicated detachment tool 100 includes the cutter unit 112 at a recessed portion 104 of a housing 111, and two sponges 103 for dirt removal which are arranged opposed to each other with a predetermined interval. A slit 105 is formed at an inner side surface portion of the recessed portion 104 of the housing 111 in accordance with a movable range of the two blades 106, 107 of the cutter unit 112.

The two blades 106, 107 of the cutter unit 112 include edge portions 106a, 107a cut in an arc shape which are formed at the upper portion, and semicircular recessed portions 106b, 107b which are formed at the middle portion, and the two blades are joined at the lower portion side by a rotational shaft 108. Note that, in the cutter unit 112, the two blades 106, 107 are connected by the rotational shaft 108 such that the two edge portions 106a, 107a form a continuous arc shape at the upper portion side and the two semicircular recessed portions 106b, 107b form a circular hole portion at the middle portion. A twist spring 109 for biasing the two blades is externally inserted and arranged on the rotational shaft 108.

If the distal end part of the communication cable 43 having the drip-proof portion 70 is inserted into the dedicated detachment tool 100 so as to be pressed from the upper portion side, incisions are formed on the heat-shrinkable tube 71 by the two edge portions 106a, 107a. At this time, the two blades 106 and 107 relatively rotate such that the upper portion side opens, and the communication cable 43 is pushed into the hole portion formed by the two recessed portions 106b, 107b.

Then, if the communication cable 43 is pulled out, the heat-shrinkable tube 71 on which incisions are formed can be detached, together with the head portion 72 having the latching portion 73, from the electric terminal portion 61. In addition, the drip-proof portion 70 is entered into the gap between the two sponges 103, thereby allowing dirt to be removed.

Note that the member for dirt removal is not limited to the sponges 103, and may be an elastic body such as a rubber wiper. Furthermore, the two dedicated tools for detachment 80, 100 according to the two configurations as described above may be integrally provided to the extracorporeal apparatus 3.

### (Third Embodiment)

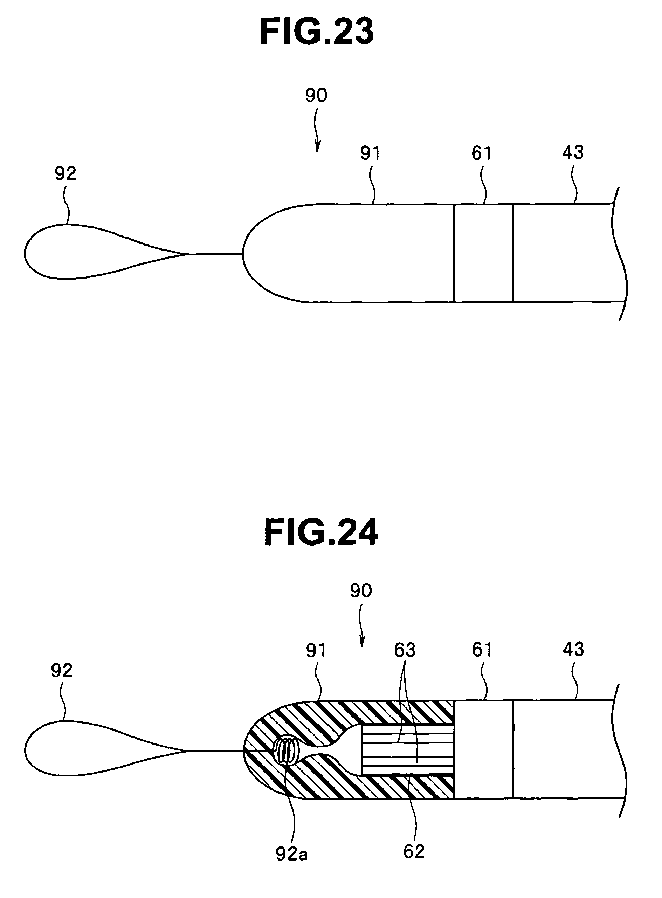

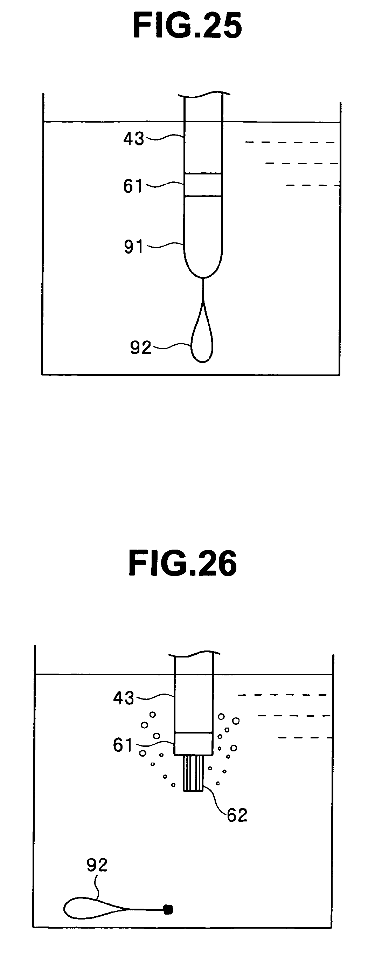

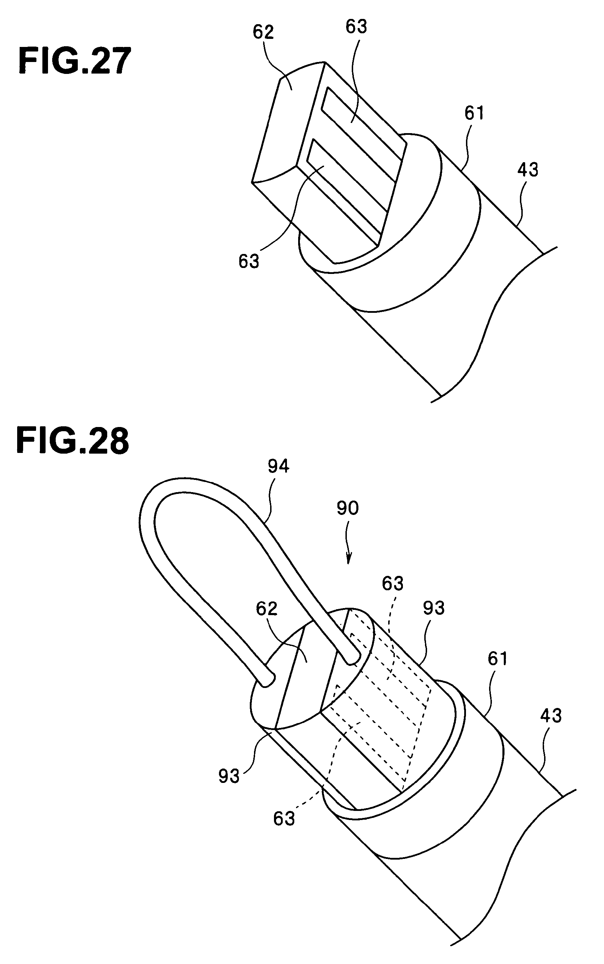

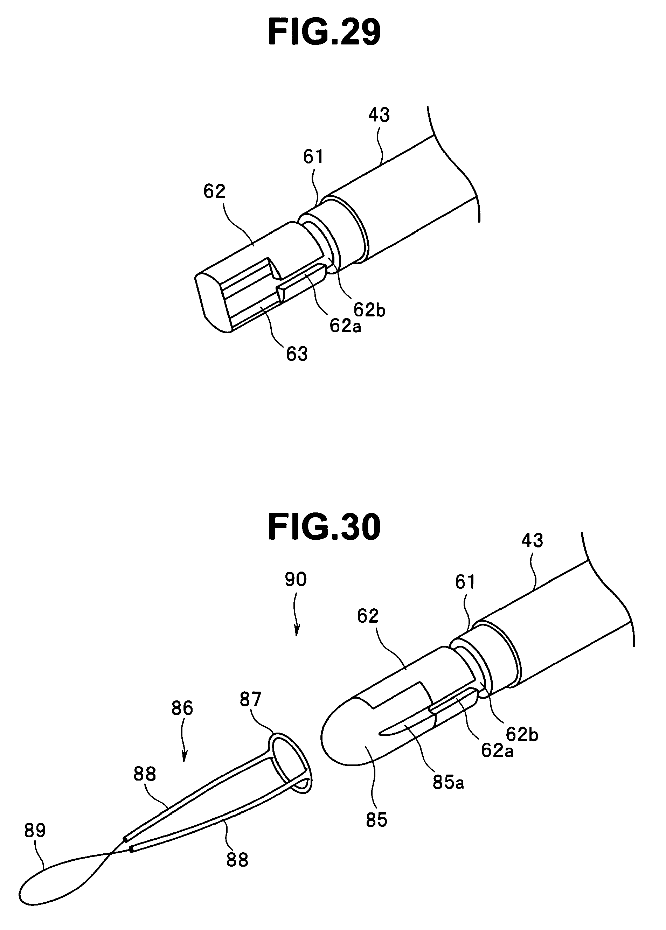

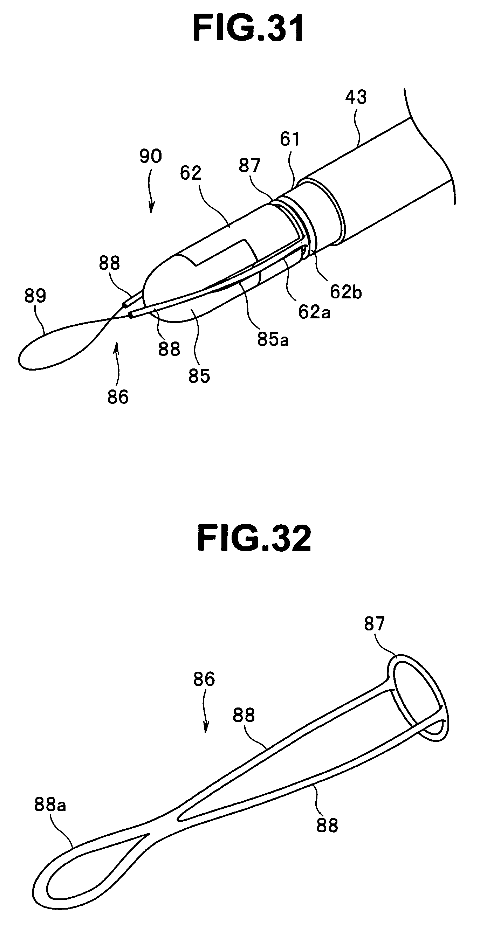

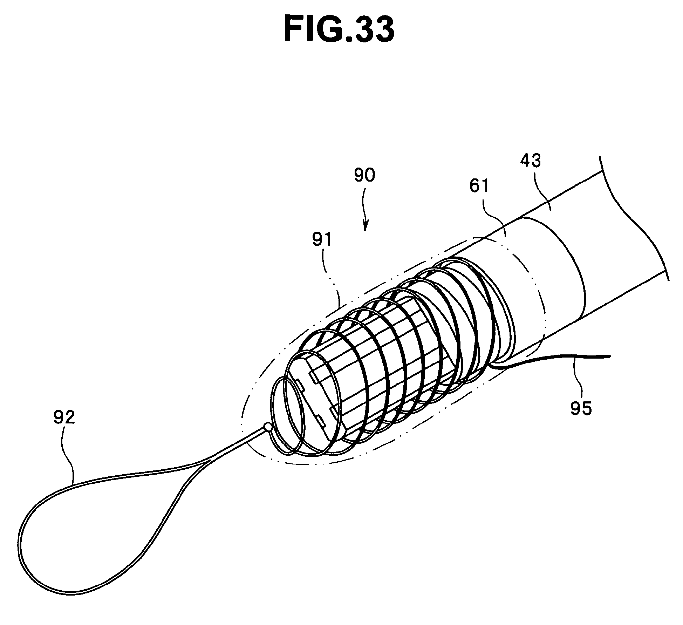

Next, with reference to FIGS. 23 to 33, the third embodiment of the endoscope system according to the present invention will be described below. Note that FIGS. 23 to 33 relate to the third embodiment of the present invention in which: FIG. 23 is a side view showing a configuration of a drip-proof portion; FIG. 24 is a cross-sectional view showing the configuration of the drip-proof portion in FIG. 23; FIG. 25 is a view showing a state where the drip-proof portion is dipped in medicinal solution; FIG. 26 is a view showing a state where the drip-proof portion is subjected to dipping processing in the medicinal solution; FIG. 27 is a perspective view showing a configuration of the electric terminal portion according to a first modified example; FIG. 28 is a perspective view showing the configuration of the drip-proof portion covering the electric terminal portion in FIG. 27; FIG. 29 is a perspective view showing a configuration of an electric terminal portion according to a second modified example; FIG. 30 is an exploded perspective view showing the configuration of the drip-proof portion covering the electric terminal portion in FIG. 29; FIG. 31 is a perspective view showing the configuration of the drip-proof portion covering the electric terminal portion in FIG. 29; FIG. 32 is a perspective view showing a modified example of an engaging portion; and FIG. 33 is a perspective view showing a configuration of a drip-proof portion covering an electric terminal portion according to a third modified example. In addition, also in the description below, the same reference numerals are attached to the same components as those in the endoscope system 1 according to the first and second embodiments, detailed descriptions thereof will be omitted, and the features thereof can be applied to the above-described embodiments.

As shown in FIGS. 23 and 24, a drip-proof portion 90 as a cover body according to the present embodiment includes a latching portion 92 formed by a ring-shaped wire, and a resin-coated portion 91 formed by spraying a biocompatible resin to the male terminal 62 of the electric terminal portion 61 and coating the male terminal 62 with the resin. Note that the latching portion 92 is configured such that the end portion thereof housed in the resin-coated portion 91 is formed in a coil shape so as not to be fallen off from the resin-coated portion 91.

The male terminal 62 of the electric terminal portion 61 is coated with resin and a plurality of electric contacts 63 on both side surfaces are also covered with the resin-coated portion 91, to be drip-proofed (prevented from adherence of dirt such as body fluid and blood, and prevented from dust).

In a case where silicone is used as the resin configuring the resin-coated portion 91, for example, the drip-proof portion 90 thus configured is dipped in a resin dissolving agent, e.g., resin dissolving agent DYNASOLVE (products of D company) in a container as easy-peel means (easy-peel portion) to resolve the resin-coated portion 91, thereby enabling the drip-proof portion 90 to be removed from the male terminal 62 of the electric terminal portion 61, as shown in FIG. 25 and 26. Note that the container having the resin dissolving agent may be provided integrally with the extracorporeal apparatus 3.

### (First Modified Example)

Note that, as shown in FIGS. 27 and 28, the drip-proof portion 90 may be configured such that a resin-coated portion 93 is provided individually on each of the both side surfaces of the male terminal 62 of the electric terminal portion 61 so as to cover the plurality of electric contacts 63, and each of the two resin-coated portions 93 contains each of the end portions of the latching portion 94 formed by a loop-shaped wire.

### (Second Modified Example)

In addition, as shown in FIGS. 29 to 31, the drip-proof portion 90 may be configured such that a resin-coated portion 85 is formed from the both side surfaces to the distal end surface of the male terminal 62 of the electric terminal portion 61 so as to cover the plurality of electric contacts 63, two long grooves 62a (only one is illustrated in the figures) are formed on the male terminal 62 of the electric terminal portion 61 in a front-back direction at side portions at point-symmetric positions with respect to the central axis, a circumferential groove 62b having a recessed shape is formed around the outer circumference so as to be connected to the proximal ends of the two long grooves 62a, and an elastic latching portion 86 configured to be mounted by engagement to the grooves 62a, 62b is provided as a separate body. Note that the resin-coated portion 85 includes two recessed groove portions 85a (only one is shown in FIGS. 30, 31) formed at side portions along the two long grooves 62a.

Furthermore, the latching portion 86 includes a resin ring portion 87 to be engaged in the circumferential groove 62b, two arm portions 88 formed to be extended from the ring portion 87 and to be engaged in the long grooves 62a and the groove portions 85a, and loop-shaped metal wire 89 extending from the two arm portions 88. Note that the entirety of the latching portion 86 may be resin-molded and provided with a loop portion 88a formed at the front of the position where the distal end parts of the two arm portions 88 are joined.

### (Third Modified Example)

Furthermore, as shown in FIG. 33, the drip-proof portion 90 may be formed such that a wire member 95 spirally wound around the male terminal 62 of the electric terminal portion 61 is arranged and the resin-coated portion 91 may be formed to cover the whole wire member 95 including the male terminal 62 of the electric terminal portion 61. Note that the wire member 95 has one end connected to the end portion of a latching portion 92 formed by a wire, inside the resin-coated portion 91, and has the other end extended from the rear part of the resin-coated portion 91.

The resin-coated portion 91 is broken by pulling the extension end of the wire member 95, thereby enabling the drip-proof portion 90 to be removed from the male terminal 62 of the electric terminal portion 61. That is, the wire member 95 configures easy-peel means (easy-peel portion) in this configuration.

The invention described above is not limited to the above embodiments but various modifications thereof are possible without departing from the gist of the invention in a practical stage. Furthermore, the above-described embodiments include inventions of various stages, and by combining a plurality of constituent components disclosed in the embodiments, inventions of various stages can also be extracted.

For example, even if some constituent components are deleted from all the constituent components shown in the above-described present embodiments, if the problem to be solved by the invention can be solved and the effects of the invention can be obtained, the configuration in which some constituent components are deleted can be extracted as an invention.

The present application is filed claiming the priority of Japanese Patent Application No. 2010-158952 filed in Japan on July 13, 2010, and the above described disclosure is incorporated by reference in the present description, claims and drawings.

## Claims

1. A medical apparatus configured to be introduced into a body, comprising:
an image pickup apparatus for picking up an image in the body;
a cable extended from the image pickup apparatus, and configured to transmit and receive a signal or power to and from an external device, the cable being pulled out from inside to outside the body through a body wall;
an electric terminal portion disposed at an end portion of the cable, which includes an electric contact and is configured to be electrically connected to the external device;
a cover body which is configured to cover at least the electric contact and to be adhered watertightly to the electric terminal portion with a predetermined strength, the cover body being grasped when the cable is pulled out from inside to outside the body; and
an easy-peel portion for detaching the cover body from the electric terminal portion.

2. The medical apparatus according to claim 1, wherein the cover body is made of an easy-peel resin, and the easy-peel portion is an easy-peel adhering portion subjected to bonding processing by taking advantage of a characteristic of easy-to-unseal resin.

3. The medical apparatus according to claim 2, wherein the easy-peel portion includes an easy-to-unseal processed part provided at a peripheral portion of the cover body.

4. The medical apparatus according to claim 3, wherein the easy-to-unseal processed part is an incision.

5. The medical apparatus according to claim 1, wherein the cover body is formed by a heat-shrinkable tube, and the easy-peel portion includes a cutter for making an incision on the heat-shrinkable tube to detach the heat-shrinkable tube from the electric terminal portion.

6. The medical apparatus according to claim 1, wherein the cover body is made of a resin, and the easy-peel portion includes a resin dissolving agent for dissolving the resin.

7. The medical apparatus according to any one of claims 1 to 6, wherein the cover body is provided with a latching portion which is configured to be able to be hooked when taken out from inside to outside the body.

8. The medical apparatus according to claim 7, wherein the cover body is provided with the latching portion configured to be detachable from the cover body.

9. A medical apparatus configured to be introduced into a body, comprising:
image pickup means for picking up an image in the body;
a cable extended from the image pickup means, and configured to transmit and receive a signal or power to and from an external device, the cable being pulled out from inside to outside the body through a body wall;
an electric terminal portion disposed at an end portion of the cable, which includes an electric contact and is configured to be electrically connected to the external device;
a cover body which is configured to cover at least the electric contact and to be adhered watertightly to the electric terminal portion with a predetermined strength, the cover body being grasped when the cable is pulled out from inside to outside the body; and
easy-peel means for detaching the cover body from the electric terminal portion.
